Europäisches Patentamt

European Patent Office

Office européen des brevets

(19)

(11) Veröffentlichungsnummer: **0 280 772 B1**

(12) # EUROPÄISCHE PATENTSCHRIFT

(45) Veröffentlichungstag der Patentschrift: **12.06.91**

(51) Int. Cl.⁵: **C07C 53/10**, C07C 51/41

(21) Anmeldenummer: 87117029.6

(22) Anmeldetag: **19.11.87**

(54) Verfahren zur Herstellung granulierter Calcium- und Magnesium-acetate bzw. deren Gemische.

(30) Priorität: **21.02.87 DE 3705618**

(43) Veröffentlichungstag der Anmeldung:
**07.09.88 Patentblatt 88/36**

(45) Bekanntmachung des Hinweises auf die
Patenterteilung:
**12.06.91 Patentblatt 91/24**

(84) Benannte Vertragsstaaten:
**AT BE CH DE FR GB IT LI NL SE**

(56) Entgegenhaltungen:
**US-A- 4 060 535**
**US-A- 4 511 485**
**US-A- 4 636 467**

(73) Patentinhaber: **KALI UND SALZ AKTIENGE-
SELLSCHAFT**
**Friedrich-Ebert-Strasse 160**
**W-3500 Kassel(DE)**

(72) Erfinder: **Kerscher, Utto, Dr.**
**Am Beissel 25**
**W-5024 Erfstadt(DE)**
Erfinder: **Straub, Otto**
**Hevinghausen 46**
**W-5203 Much(DE)**
Erfinder: **Alsdorf, Hermann, Dr.**
**Gross Odescheid 120**
**W-5063 Much(DE)**

(74) Vertreter: **Werner, Hans-Karsten, Dr. et al**
**Patentanwälte von Kreisler-Selting-Werner
Schönwald-Fues-Dallmeyer Hilleringmann,
Deichmannhaus
W-5000 Köln 1(DE)**

EP 0 280 772 B1

## Beschreibung

Die Erfindung betrifft ein Verfahren zur Herstellung granulierter Calcium-und Magnesiumacetate bzw. deren Gemische durch Umsetzung der Erdalkalioxide bzw. -hydroxide mit Essigsäure.

Im Hinblick auf die Umweltbelastung durch chloridische Taumittel, wie NaCl, $CaCl_2$ und $MgCl_2$ im Winterdienst für Straßen und Fahrbahnen wurde als alternatives Taumittel Calcium-Magnesiumacetat (CMA) mit Erfolg getestet und erprobt. Dies wird in diversen wissenschaftlichen Berichten referiert. Wenngleich CMA eine schlechtere Auftauwirkung im Vergleich zu beispielsweise NaCl oder $CaCl_2$ besitzt, was durch eine größere Einsatzmenge kompensiert würde, so sind doch die antikorrosiven Eigenschaften und die gute biologische Abbaubarkeit in Boden und Wasser sehr vorteilhaft zu bewerten. Die antikorrosiven Eigenschaften und die günstigen Umwelteigenschaften des CMA gestatten es auch, dieses Produkt insbesondere als Taumittel auf Flugplätzen einzusetzen, wo die Verwendung des üblicherweise als Taumittel eingesetzten NaCl u./o. $CaCl_2$ nicht möglich ist.

Zweckmäßigerweise werden Taumittel als nicht-staubendes Granulat verwendet, um Wehverluste und Staubbelästigungen zu vermeiden. Nur in granulierter Form läßt sich das Taumittel mit den üblichen Streugeräten ausbringen.

In einem Forschungsbericht, herausgegeben vom Bundesministerium für Bauten und Technik, Wien, Straßenforschung, Heft 262, ist zu entnehmen, daß sich bei der Verwendung von CMA als Taumittel deutliche umwelt- und pflanzenfreundliche Eigenschaften erkennen lassen.

Die Herstellung des CMA im Labormaßstab ist in dieser wissenschaftlichen Veröffentlichung beschrieben. Danach wurde versucht, gebrannten Dolomit (Ca-Mg-oxid) bei einer Temperatur von 80° C in konzentrierter Essigsäure zu lösen, um beim Abkühlen des Gemisches CMA zu erhalten. Es war dieser Veröffentlichung zufolge jedoch nicht möglich, größere Mengen gebrannten Dolomit in Lösung zu bringen. Nur durch Auflösen von gebranntem Dolomit in verdünnter Essigsäure (1:1) und nachfolgender Einengung der Lösung wurde eine größere Menge CMA erhalten, das für die dort beschriebenen Untersuchungen verwendet wurde.

Ein weiteres Herstellungsverfahren für CMA ist in der US-PS 4 606 823 beschrieben. Danach kann CMA durch Umsetzung von gelöschtem oder ungelöschtem Dolomit-Kalk mit einem Essigsäure/Wasser-Gemisch erhalten werden, wobei der Gesamtwassergehalt des Gemisches kritisch ist und im Bereich von 3,3 bis 7,0 Mol/Mol Calcium-Magnesiumacetat liegen soll. Entsprechend den Herstellungsbeispielen der US-PS 4 606 823 wird das erhaltene Produkt bis zur Gewichtskonstanz bei 100° C getrocknet. Eigene Untersuchungen der Anmelderin haben gezeigt, daß Reste von anhaftender Essigsäure und insbesondere die zum Teil in dem Kristall gebundene Essigsäure bei dieser Trockentemperatur unter normalen Bedingungen nicht zu entfernen ist. In dem auf diese Weise getrockneten Produkt sind deshalb noch unerwünschte und geruchlich wahrnehmbare Mengen an Essigsäure enthalten.

Weiterhin wurde jetzt gefunden, daß die Umsetzung von Calcium- und/oder Magnesiumoxiden mit wäßriger verdünnter Essigsäure, mit einem Essigsäuregehalt von weniger als 85 % nur unvollständig verläuft. Je höher der Wasseranteil der verdünnten Essigsäure ist, umso mehr kommt es zur Bildung einer schmierig-klebrigen Mischphase. Es kann keine einwandfreie Durchmischung mehr erfolgen und es kann letzten Endes wegen des zu großen Widerstands der klebrigen Produkte zum Stillstand des Mischwerkzeugs kommen.

Aufgabe der Erfindung war es, ein Verfahren zu entwickeln, wonach granulierte Calcium- und Magnesiumacetate bzw. deren Gemische sicher und reproduzierbar in großtechnischem Maßstab hergestellt werden können und das granulierte Produkt möglichst geringe Mengen in kaltem Wasser unlösliche Anteile enthält.

Es wurde gefunden, daß diese Aufgabe dadurch gelöst werden kann, daß man Calcium- und/oder Magnesiumoxid bzw. Calcium-Magnesiumoxide oder -hydroxide mit einem geringen Überschuß an 85 - 100 %iger Essigsäure, Eisessig bzw. Essigsäureanhydrid in einem Mischreaktor unter Rückflußkühlung umsetzt, gleichzeitig granuliert und anschließend in einem Bewegungstrockner die restliche Essigsäure zusammen mit dem Wasser aus dem Reaktionsprodukt entfernt.

Als Calcium- und/oder Magnesiumoxide bzw. -hydroxide können vorzugsweise gebrannter Dolomit (Dolomit-Weichbrannt), gebrannter und gelöschter Kalk (CaO oder $Ca(OH)_2$) und Magnesiumoxid bzw. -hydroxid eingesetzt werden. Die 85 - 100 %ige, vorzugsweise 95 - 100 %ige, und besonders bevorzugte 99 %ige Essigsäure bzw. das Essigsäureanhydrid werden mit einem Überschuß von 2 - 10 %, vorzugsweise 5 %, bezogen auf die stöchiometrische Menge, eingesetzt. Die Überschußmenge an 85 - 100 %iger Essigsäure bzw. Essigsäureanhydrid ist notwendig, um eine möglichst vollständige Umsetzung der Erdalkalioxide bzw. -hydroxide zu erreichen. Als Mischreaktor eignen sich übliche Zwangsmischer, insbesondere Pflugscharmischer, in welchem die Umsetzung im Laufe von 2 - 30 Minuten, vorzugsweise 15 Minuten, unter Rückflußkühlung der Essigsäure vorgenommen wird. das CMA wird dabei unmittelbar in Form von Granalien erhalten. Getrocknet wird das CMA am wirkungsvollsten in einem Wirbelschichttrockner bei einer Temperatur von 110 160° C, vorzugsweise bei 140 - 150° C.

Zur Herstellung der Acetate (CMA) wird das pulverförmige Erdalkalioxid oder -hydroxid in dem Mischreaktor mit einer Rückfluß-Kühlvorrichtung vorgelegt und die stöchiometrische Menge an 85 - 100 %iger Essigsäure sowie der geringe Überschuß im Laufe von etwa 15 Minuten zugefügt. Die durch die Reaktionswärme siedende Essigsäure wird durch die Rückflußkühlung kondensiert und nach einer etwa 5-minütigen Nachreaktionszeit verbleibt ein granuliertes Acetat. Die überschüssige Essigsäure und das bei der Reaktion entstehende Wasser werden vorzugsweise in einem Wirbelschichttrockner bei einer Temperatur von 140 - 150° C entfernt. Die ausgetriebene Essigsäure wird in einem nachgeschalteten Wasser-Wäscher aufgefangen und kann gegebenenfalls als verdünnte Essigsäure zur Herstellung von Acetat-Lösung verwendet werden.

Nach dem Trockenvorgang wird zunächst ein vollkommen geruchsneutrales Acetat-Granulat von unterschiedlicher Korngröße erhalten. Es wird daher anschließend das Nutzkorn durch Sieben herausgetrennt. Der Anteil des Nutzkorns mit einer Korgröße von 0,5 - 4,0 mm beträgt etwa 70 %. Das Überkorn kann gemahlen und zusammen mit dem Unterkorn dem Prozess wieder zugeführt werden. Die Korngröße kann durch Variation der Reaktionszeit des Acetat-Gemisches beeinflußt werden. So wird durch eine langsame Zudosierung der Essigsäure bzw. des Essigsäureanhydrids ein feineres Granulat erhalten, und bei schnellerem Zufügen der Essigsäure oder -anhydrids eine gröbere Granulierung erreicht.

Die so hergestellten Acetate sind als Taumittel in Form von Granalien wie auch als konzentrierte Lösung einsetzbar. Sie sind nicht korrosiv und nicht ätzend, sie erweisen sich als umweltfreundlich und geruchsneutral. Die Granalien sind nahezu rückstandslos in kaltem Wasser löslich.

Mit den nachfolgenden Beispielen soll das erfindungsgemäße Verfahren näher erläutert werden.

Beispiel 1

15 kg Dolomit-Weichbrannt ( 56 % CaO, 37 % MgO ) werden in einem Pflugscharmischer mit einer Rückfluß-Kühlvorrichtung vorgelegt und im Laufe von 15 Minuten werden bei laufendem Mischwerkzeug 36,2 kg Eisessig ( = 5 % Überschuß ) zugefügt. Nach 5-minütiger Nachreaktion ist die Umsetzung beendet. Es werden 45,7 kg granuliertes CMA erhalten, das noch 3 Gew-% Essigsäure enthält. In einem Wirbelschichttrockner wird bei einer Temperatur von 140° C im Laufe von 30 Minuten die Rest-Essigsäure und Restfeuchte ausgetrieben und in einem nachgeschalteten Wasser-Wäscher aufgefangen. Von dem getrockneten Produkt wird das Über- und Unterkorn durch Sieben abgetrennt. Als Nutzkorn mit einer Korngröße von 0,5 - 4,0 mm werden 31 kg CMA erhalten. Diese Ausbeute entspricht 70 % der Theorie. Das Produkt enthält < 3 % wasserunlösliche Anteile.

Beispiel 2

15 kg gebrannter Kalk ( 94 % CaO, 1,2 % MgO ) werden analog dem Beispiel 1 mit 32,2 kg Eisessig umgesetzt, getrocknet und gesiebt. Es werden 28,7 kg granuliertes CMA mit einer Korngröße von 0,5 - 4,0 mm erhalten.

Beispiel 3

15 kg Magnesiumhydroxid ( 40 % Mg ) werden analog dem Beispiel 1 mit 26,4 kg Essigsäureanhydrid umgesetzt, getrocknet und gesiebt. Es werden 23,0 kg granuliertes Mg-acetat mit einer Korngröße von 0,5 - 4,0 mm erhalten.

Beispiel 4

13,5 kg Rückgut ( aufgemahlenes Über- und Unterkorn, das entsprechend dem Beispiel 1 erhalten wurde ), werden mit 10,7 kg Dolomit-Weichbrannt vermischt und analog dem Beispiel 1 mit 25,8 kg Eisessig umgesetzt, getrocknet und gesiebt. Es werden 34,0 kg granuliertes CMA mit einer Korngröße von 0,5 - 4,0 mm erhalten.

Beispiel 5 (Vergleichsbeispiel)

Analog dem Beispiel 1 werden 15 kg Dolomit-Weichbrannt im Laufe von 30 Minuten mit 45,6 kg 80 %iger wäßriger, verdünnter Essigsäure umgesetzt. Es bildet sich eine klebrig-teigige Masse, die eine einwandfreie Durchmischung unmöglich macht und das Mischwerkzeug zum Stillstand bringt. Die Trocknung dieses Produkts im Wirbelschichttrockner ist wegen der schlechten Granulierung und der extrem ungleichmäßigen Körnung wesentlich schwieriger als bei den erfindungsgemäßen Beispielen. Nach dem Sieben beträgt die Nutzkornausbeute mit einer Korngröße von 0,5 - 4,0 mm < 20 %. das Produkt enthält > 12 % wasserunlösliche Anteile.

Beispiel 6 (Vergleichsbeispiel)

Analog dem Beispiel 4 werden 13,5 kg Rückgut mit 10,7 kg Dolomit-Weichbrannt vermischt und analog dem Beispiel 1 mit 32,2 kg 80 %iger, verdünnter wäßriger Essigsäure im Laufe von 20 Minuten umgesetzt, getrocknet und gesiebt. Bei dem erhaltenen granulierten CMA mit einer Korngröße von 0,5 - 4,0 mm beträgt die Ausbeute weniger als 50 %. Das Produkt enthält > 8 % wasserunlösliche Anteile.

## Ansprüche

1. Verfahren zur Herstellung granulierter Calcium- und Magnesiumacetaten bzw. deren Gemische durch Umsetzung der Erdalkalioxide bzw. -hydroxide mit Essigsäure, **dadurch gekennzeichnet, daß** man Calcium- und/oder Magnesiumoxid bzw. Calcium-Magnesiumoxide oder -hydroxide mit einem geringen Überschuß an 85 - 100 %iger Essigsäure, Eisessig bzw. Essigsäureanhydrid in einem Mischreaktor unter Rückflußkühlung umsetzt, gleichzeitig granuliert und anschließend in einem Bewegungstrockner die restliche Essigsäure zusammen mit dem Wasser aus dem Reaktionsprodukt entfernt.

2. Verfahren nach Anspruch 1, **dadurch gekennzeichnet, daß** man als Calcium- u./o. Magnesiumoxid bzw. Calcium-Magnesiumoxide oder -hydroxide Calciumoxid (gebrannten Kalk), Magnesiumoxid, gebrannten Dolomit u./o. deren Gemische und Calciumhydroxid, Magnesiumhydroxid, gebrannten und gelöschten Dolomit (Dolomit-Kalk-hydrat) u./o. deren Gemische einsetzt bzw. Gemische der genannten Oxide und Hydroxide einsetzt.

3. Verfahren nach Ansprüchen 1 und 2, **dadurch gekennzeichnet, daß** man die Essigsäure mit einem stöchiometrischen Überschuß von 2 - 10 %, vorzugsweise 5 % einsetzt.

4. Verfahren nach einem der Ansprüche 1 bis 3, **dadurch gekennzeichnet, daß** man 95 - 100 %ige Essigsäure einsetzt.

5. Verfahren nach einem der Ansprüche 1 bis 4, **dadurch gekennzeichnet, daß** man die restliche Essigsäure und das Wasser bei 110 - 160 °C entfernt.

6. Verfahren nach einem der Ansprüche 1 bis 5, **dadurch gekennzeichnet, daß** man die Reaktion in einem Zwangsmsicher mit Rückflußkühler, vorzugsweise einem Pflugscharmischer, durchführt.

7. Verfahren nach einem der Ansprüche 1 bis 6, **dadurch gekennzeichnet, daß** man als Bewegungstrockner einen Wirbelschichttrockner einsetzt.

## Claims

1. A process for preparing granulated calcium and magnesium acetates or mixtures thereof by reacting the alkaline earth metal oxides and/or hydroxides with acetic acid, **characterized in that** calcium and/or magnesium oxide or calcium-magnesium oxides or hydroxides are reacted with a low excess amount of a 85 to 100% acetic acid, glacial acetic acid and/or acetic anhydride in a mixer-reactor with reflux condensation and then the residual acetic acid is removed together with the water from the reaction product in an agitated dryer.

2. The process according to claim 1, **characterized in that,** as the calcium and/or magnesium oxide or calcium-magnesium oxides or hydroxides, there are employed calcium oxide (caustic lime, quicklime), magnesium oxide, burnt dolomite and/or mixtures thereof and calcium hydroxide, magnesium hydroxide, burnt and hydrated (slaked) dolomite (dolomite-lime hydrate) and/or mixtures thereof or mixtures of said oxides and hydroxides, respectively.

3. The process according to claims 1 and 2, **characterized in that** the acetic acid is employed in a stoichometric excess of from 2 to 10%, and preferably of 5%.

4. The process according to any of claims 1 to 3, **characterized in that** a 95-100% acetic acid is employed.

5. The process according to any of claims 1 to 4, **characterized in that** the residual acetic acid and the water are removed at from 110 °C to 160 °C.

6. The process according to any of claims 1 to 5, **characterized in that** the reaction is carried out in a forced circulation mixer, and preferably in a ploughshare mixer.

7. The process according to any of claims 1 to 6, **characterized in that,** as the agitated dryer, there is employed a fluidized bed dryer.

## Revendications

1. Procédé d'obtention d'acétates de calcium et de magnésium ou de leurs mélanges, sous forme granulée, par réaction de l'oxyde ou hydroxyde alcalino-terreux avec l'acétate acétique,
caractérisé en ce qu'on fait réagir de l'oxyde ou de l'hydroxyde de calcium et/ou de magnésium ou de l'oxyde ou hydroxyde de calcium et magnésium avec un léger excès d'acide acétique à 85-100 %, d'acide acétique glacial, ou d'anhydride acétique dans un réacteur-mélangeur avec refroidissement à reflux, on procède à la granulation simultanément, et ensuite on sépare du produit de la réaction l'acide acétique résiduel, en même temps que l'eau, dans un séchoir à agitation.

2. Procédé selon la revendication 1, caractérisé en ce qu'on utilise, comme oxyde ou hydroxyde de calcium et/ou de magnésium ou de calcium et magnésium, de l'oxyde de calcium (chaux calcinée), de l'oxyde de magnésium, de la dolomie calcinée et/ou leurs mélanges, et de l'hydroxyde de calcium, de l'hydroxyde de magnésium, de la dolomie calcinée et éteinte (hydrate de dolomie-chaux) et/ou leurs mélanges, ou des mélanges desdits oxydes et hydroxydes.

3. Procédé selon les revendications 1 et 2, caractérisé en ce qu'on utilise l'acide acétique avec un excès sur la stoechiométrie de 2 à 10 %, de préférence 5 %.

4. Procédé selon l'une des revendications 1 à 3, caractérisé en ce qu'on utilise de l'acide acétique à 95-100 %.

5. Procédé selon l'une des revendications 1 à 4, caractérisé en ce qu'on enlève l'acide acétique résiduel et l'eau à 110-160° C.

6. Procédé selon l'une des revendications 1 à 5, caractérisé en ce qu'on réalise la réaction dans un mélangeur à mélange forcé, à réfrigérant à reflux, de préférence un mélangeur à socs.

7. Procédé selon l'une des revendications 1 à 6, caractérisé en ce qu'on utilise un séchoir à lit fluidisé comme séchoir à agitation.